# EUROPEAN PATENT SPECIFICATION

(11) **EP 0 367 757 B1**
(45) Date of publication and mention of the grant of the patent: **02.11.1994**
(21) Application number: 87903666.3
(22) Date of filing: 22.05.1987
(51) Int. Cl.: A61K 39/116, A61K 37/20, A61K 39/108, A61K 39/39, A61K 39/02, A61K 39/102, A61K 39/10, A61K 39/112, A61K 39/106, A61K 39/104

(54) **CO-VACCINATION USING NON-O-CARBOHYDRATE SIDE-CHAIN GRAM-NEGATIVE BACTERIA PREPARATION**
GEMEINSAME IMPFUNG UNTER VERWENDUNG EINER PRAPARATION GRAM-NEGATIVER BAKTERIEN OHNE O-SPEZIFISCHEN KOHlEHYDRATSEITENKETTEN
CO-VACCINATION UTILISANT UNE PREPARATION DE BACTERIES GRAM-NEGATIVES SANS CHAINES LATERALES O-HYDRATE DE CARBONE

(30) Priority: 23.05.1986 US 866451
(43) Date of publication of application: 16.05.1990
(73) Proprietor: SANOFI ANIMAL HEALTH, Inc., Overland Park, Kansas 66210 (US)
(72) Inventor: NELSON, Ralph, Shawnee, KS 66203 (US); SCHLINK, Gerald, Irwin, MI 64759 (US)
(74) Representative: Gillard, Marie-Louise
(86) International application number: US8701222
(87) International publication number: WO8707148

(56) References cited:
- EP-A- 0 089 283
- EP-A- 0 158 282
- WO-A-80/02113
- GB-A- 1 085 956
- GB-A- 2 076 287
- US-A- 4 016 253
- US-A- 4 167 560
- US-A- 4 455 142
- US-A- 4 469 672
- BIOLOGICAL ABSTRACTS, vol. 74, no. 6, 1982, Philadelphia, PA (US); M.I. MARKS et al., p. 4098, no. 39459#
- THE LANCET, vol. II, 13 July 1985, The Lancet Ltd.; J.-D. BAUMGARTNER et al., pp. 59-63#
- INFECTION & IMMUNITY, vol. 45, no. 3, September 1984, American Society for Microbiology; L.M. MUTHARIA et al., pp. 631-636#
- INFECTION & IMMUNITY, vol. 46, no. 3, December 1984, American Society for Microbiology; M.J. NELLES et al., pp. 677-681#
- BIOLOGICAL ABSTRACTS, vol. 82, no. 9, 1986, Philadelphia, PA (US);B.W. FENWICK et al., p. AB-466, no. 82938#

## Description

### Field of the Invention

This invention relates to an improved composition for co-vaccination of animals, including mammals and birds, against gram-negative organisms and the diseases caused thereby. More particularly, the invention concerns a co-vaccine that employs a bacterial lipopolysaccharide (LPS) fraction devoid of O-carbohydrate side-chains, exemplified by E. coli J5 and mutants thereof, with a bacterin directed to one or more gram-negative organisms for the immunological protection of an animal against gram-negative organisms, and the diseases caused by these organisms.

### Background of the Invention

Gram-negative bacteria have similar LPS structures. Some mutant gram-negative bacterial strains lack the O-carbohydrate side-chains normally associated with gram-negative bacteria. These mutant organisms lack pili and outer antigens which are normally associated with the LPS membrane leaving LPS and other core antigens exposed.

Escherichia coli strain J5 is a well known example of a genetically stable gram-negative bacterial species having LPS and other core antigens exposed. Other gram-negative bacteria of different species, such as Salmonella enteritidis, ATCC No. 53000, described in European Patent Application 0158282, also lack the O-carbohydrate side-chains (also known as "K antigens"). The European Patent Application teaches a method of preparing non-O-carbohydrate side-chain gram-negative bacteria.

EP-A-0089283 describes the preparation and use of inactive bacterial toxoids derived from ADP-ribosylating toxins which are useful as immunogene.

### Summary of the Invention

In accordance with the present invention, a co-vaccine suitable for administration against gram-negative organisms which contains an effective dose of gram-negative bacterial cells, as source of bacterial lipopolysaccharide devoid of O-carbohydrate side-chains, bacterins of one or more gram-negative organisms, and optionally a pharmaceutically acceptable carrier, is disclosed Administration of the co-vaccine is achieved by the co-injection of the bacterial lipopolysaccharide devoid of O-carbohydrate side-chains and the bacterins.

Also disclosed is a method of enhancing the immune response, and thus protecting an animal against diseases caused by gram-negative organisms by co-injecting the animal with an effective amount of bacterial lipopolysaccharide (LPS) devoid of O-carbohydrate side-chains in combination with bacterins of one or more gram-negative organisms.

### Detailed Description of the Invention

The present invention relates to the co-administration of an effective amount of gram-negative bacterial cells, as source of bacterial lipopolysaccharide devoid of O-carbohydrate side-chains in conjunction with a vaccine specifically directed to each gram-negative organism to which immunization is desired. It has been found that bacterial lipopolysaccharide devoid of O-carbohydrate side-chains is an effective immunomodulator when used in conjunction with gram-negative bacterins.

It has been found that the process of co-injection of bacterial LPS devoid of O-carbohydrate side-chains in combination with gram-negative bacteria provides an advantage over gram-negative bacterin preparations used alone. The process of co-injection encompasses contemporaneous administration.

The co-vaccine of the present invention may optionally be administered in admixture with a pharmacologically acceptable carrier prior to administration to an animal. Pharmacologically acceptable carriers for the invention are those usually employed in vaccines such as aqueous and oil based carriers and includes slow release antigen/adjuvant combinations. Exemplary of components in such carriers are saline, aluminum hydroxide gel, and carboxypolymethylene.

A source of bacterial lipopolysaccharide devoid of O-carbohydrate side-chains is required. E. coli strain J5, or mutants thereof, is exemplary of a gram-negative organism having exposed LPS and other core antigens. E. coli strain J5 whole cells are a preferred source of gram-negative bacterial cells, as source of bacterial lipopolysaccharide devoid of O-carbohydrate side-chains.

E. coli strain J5, i.e., ATCC No. 39355, can be cultivated in suitable growth media such as brain heart infusion or tryptic soy broth. Either enriched or minimal nutrient media can be used and the culture may be grown in glass containers or fermentors. A preferred medium is Trypticase Soy Broth (Difco Laboratories, Detroit, MI). Frozen or lyophilized J5 cultures can be used to inoculate the media.

The size of an inoculum should not be less than 0.2 percent v/v of the total volume. Growth of the organism is monitored by utilization of sugars, change in pH units and a change in the absorbance of the culture.

Gram-negative bacterial cells devoid of O-carbohydrate side-chains and bacterins of the present invention may be live or inactivated, and may be used in any combination thereof. A preferred source of bacterial lipopolysaccharide devoid of O-carbohydrate side-chains is inactivated cells. It is also preferred to use inactivated vaccines.

Gram-negative bacterial cells devoid of O-carbohydrate side-chains can be inactivated by boiling or treatment with anti-bacterial agents such as formaldehyde (0.2 percent v/v), beta-propriolactone, or antibiotics. The preferred method to inactivate the cells is with formaldehyde. The cell culture can then be optionally concentrated and/or washed to remove media components. Washed and concentrated cells devoid of O-carbohydrate side-chains are preferred.

The cell culture is typically concentrated from 5-50 percent by volume. One method to concentrate cells is the hollow-fiber method (Amicon Corporation, Danvers, MA). This method utilizes a hollow-fiber containing cartridge which includes a matrix of fibers through which the sample is pumped.

The preferred mode employs E. coli J5 cells, washed and concentrated to 2-3 x 10¹⁰ cfu/ml as determined optically. This corresponds to an approximate twenty-fold dilution of a J5 cell culture.

Bacterins of various types of gram-negative organisms are useful in the present invention. Specific examples of gram-negative bacterin strains are the following:
Pasteurella multocida, P. hemolytica, Escherichia coli, Bordetella bronchiseptica, Salmonella typhimurium, S. choleraesuis, S. dublin, Pseudomonas aeruginosa, Haemophilus pleuropneumoniae, H. parasuis, H. sommnus, Moraxella bovis, Treponema hyodysenteriae, Campylobacter sputurum, C. hyointestinalis, , Leptospira canicola, L. grippotyphosa, L. hardjo, L. icterohaemorrhagiae, L. pomoma, and L. bratislava.
Bacterins useful in the present invention are prepared according to techniques known per se.

In the preparation of the compositions of the invention, the bacterins are preferably thoroughly mixed with the bacterial lipopolysaccharide devoid of O-carbohydrate side-chains. The admixture of the bacterin or bacterins with gram-negative bacterial cells, as source of bacterial lipopolysaccharide devoid of O-carbohydrate side-chains can occur during the formulation of the bacterin or after the bacterin itself has been prepared.

The gram-negative bacterial cells, as source of bacterial lipopolysaccharide devoid of O-carbohydrates and bacterin is co-administered either undiluted or diluted with a pharmacological saline solution. Significant favorable results have been obtained with dilution values of up to about 1:25 with a number of pathogens tested. In numerous experiments undiluted or diluted solutions of about 1:5 have given very favorable results. Each milliliter of vaccine preparation preferably contains 6 - 600 x 10⁸ cfu/ml of E. coli.

The co-vaccine composition can be used to enhance the immune response, and thus protect animals prior to infection of the animals with the gram-negative pathogen for which the inoculation is prepared. The co-vaccine can also be used to stimulate the immune response, and thus protect animals currently infected with the gram-negative pathogen for which the inoculation is prepared.

This invention can be used with animals having an antigen/antibody immune response system. Specific animals in which the invention can be used include such domestic mammals as cattle, sheep, goats, pigs, dogs, cats, and horses as well as poultry animals. An approximate typical dose is .5 - 1.5 ml for poultry, 1.0 - 3 ml for pigs and 2 - 4 ml for cattle.

The following Examples are used to illustrate the invention further but should not be deemed to limit it in scope.

### Example 1 - Production of J5 Bacterin

E. coli J5 Bacterin is produced by inoculating media with actively growing seed (ATCC No. 39355). During the growth phase, the temperature is kept at 37°C ± 2°C and the pH is held constant at 7.0-7.3. The growth is monitored and maintained at a pH of 7.0-7.3 by the addition of 5 N sodium hydroxide. Dextrose is added as a sterile 50% solution to obtain maximum growth. At the end of the growth period the bacterin is inactivated with formaldehyde. After inactivation, tests are run to keep the free formaldehyde level below 0.2%.

### Example 2 - J5 and Pasteurella Multocida

An E. coli J5 culture is grown and inactivated with formaldehyde as in Example 1. It is then concentrated to approximately 5-10% of its original volume and washed with 3 volumes of physiological saline solution.

The inactivated, concentrated E. coli J5 Bacterin is then well combined with Midcon Labs' Pasteurella Multocida (PM) bacterin in the following proportions:

| | |
|---|---|
| 97.5 ml. PM Bacterin | (25% solution) |
| 2.5 ml. J5 | |
| 95.0 ml. PM Bacterin | (50% solution) |
| 5.0 ml. J5 | |

The vaccine preparations are administered subcutaneously or intramuscularly, undiluted or in a 1:5 dilution. The diluent is sterile phosphate buffered saline solution.

The results of comparative tests of the Midcon Labs' PM bacterin, E. coli J5 bacterin, and a combination of E. coli J5 bacterin with the PM bacterin appear in Table 1. USDA Pasteurella multocida (PM) Standard Reference Bacterin, IRP 248 and PM Challenge Culture, IRP 255 is used in the testing as per USDA test protocols.

**TABLE 1**

| DILUTION | USDA PM STANDARD | MIDCON PM STANDARD | MIDCON PM WITH 25% J5 | MIDCON PM WITH 50% J5 | 25% J5 ONLY | UNVACCINATED CONTROLS |
|---|---|---|---|---|---|---|
| A. UNDILUTED | #101⁺ | #201 | #301 | #401 | #501 | #601 |
| | 12/20* | 11/20 | 17/19 | 13/19 | 4/20 | 0/20 |
| B. 1:5 | #102 | #202 | #302 | #402 | #502 | #602 |
| | 1/20 | 1/20 | 4/20 | 1/20 | 0/20 | X |

| | | | | | | |
|---|---|---|---|---|---|---|
| * No. Survivors/No. Challenged | | | | | | |
| ⁺ Cage No. | | | | | | |
| X No Mice in This Group | | | | | | |

### Example 3 - J5 and Salmonella Choleraesuis

A vaccine is prepared with E. coli J5 bacterin and Midcon Labs' Salmonella choleraesuis Bacterin, as in the procedures of Example 2.

The comparative test results of the use of either bacterin alone and the co-vaccine preparation of E. coli J5 bacterin and Salmonella choleraesuis bacterin appear in Table 2. The challenge culture is Salmonella choleraesuis, USDA IRP 224.

**Table 2¹**

| DILUTION | SALMONELLA CHOLERAESUIS BACTERIN | SALMONELLA CHOLERAESUIS W/25% J-5 | SALMONELLA CHOLERAESUIS W/50% J-5 | 25% J-5 ONLY | 50% J-5 ONLY | UNVACCINATED CONTROLS |
|---|---|---|---|---|---|---|
| UNDILUTED | #201⁺ | #301 | #401 | #501 | #601 | #701 |
| | 11/20* | 15/20 | 12/20 | 3/20 | 4/20 | 0/20 |
| 1:5 | #202 | #302 | #402 | #502 | #602 | #702 |
| | 4/20 | 10/20 | 5/20 | 1/20 | 1/20 | X |

| | | | | | | |
|---|---|---|---|---|---|---|
| * No. Survivors/No. Challenged | | | | | | |
| + Cage No. | | | | | | |
| ¹ No USDA standard bacterin available. | | | | | | |
| X No Mice in This Group | | | | | | |

### Example 4 - J5 and E. coli

A vaccine is prepared with E. coli J5 bacterin and E. coli Bacterin Sero Type 987p, as in the procedures of Example 2.

Table 3 shows the results of tests of the use of either bacterin alone and the co-vaccine preparation of E. coli J5 bacterin and E. coli bacterin. A further dilution of the vaccine is tested at 1:25 as well.

**Table 3¹**

| DILUTION | E COLI BACTERIN | E COLI W/25% J-5 | E COLI W/50% J-5 | 25% J-5 ONLY | 50% J-5 ONLY | UNVACCINATED CONTROLS |
|---|---|---|---|---|---|---|
| UNDILUTED | #201⁺ | #301 | #401 | #501 | #601 | #701 |
| | 12/20* | 20/20 | 20/20 | 14/20 | 13/20 | 0/20 |
| 1:5 | #202 | #302 | #402 | #502 | #602 | #702 |
| | 6/20 | 20/20 | 18/20 | 8/20 | 10/20 | X |
| 1:25 | #203 | #303 | #403 | #503 | #603 | #703 |
| | 2/20 | 8/20 | 5/20 | 2/20 | 3/20 | X |

| | | | | | | |
|---|---|---|---|---|---|---|
| * No. Survivors/No. Challenged | | | | | | |
| ⁺ Cage No. | | | | | | |
| ¹ No USDA standard available. | | | | | | |
| X No Mice in This Group | | | | | | |

### Example 5

8 ml of Salmonella typhimurium Standard Reference Bacterin NVSL #81 IRP STB Serial 1 is mixed with 2 ml of saline, undiluted E. coli J5, E. coli J5 diluted 1:10, E. coli J5 diluted 1:100 and E. coli J5 diluted 1:1000. The admixture is then further diluted 1:5 and 1:25 with saline.

20 8 week old White Swiss Webster mice from SASCO, Omaha, NE are used for each dilution. The mice are vaccinated with 0.1 ml IP twice 2 weeks apart. 14 days after the second vaccination, the mice are challenged with 0.25 ml of a 10⁴ dilution of S. typhimurium.

Table 4 shows the results of the experiment S. typhimurium bacterin alone or in combination with E. coli J5 at various dilutions.

**Table 4**

| Dilution of S. typhimurium/E. coli J5 Bacterin Tested | | |
|---|---|---|
| VACCINE | 1:5 | 1:25 |
| J-5 Undiluted | 2/20* | 4/20 |
| J-5 1:10 | 1/20 | 4/20 |
| J-5 1:100 | 3/17 | 6/20 |
| J-5 1:1000 | 2/20 | 9/20 |
| No J-5 | 2/18 | 6/14 |

| | | |
|---|---|---|
| * No. of Dead/No. Challenged | | |

### Example 6

8 ml of Midcon Labs' Pasteurella multocida is mixed with 2 ml of phosphate buffered saline, undiluted E. coli J5, E. coli J5 diluted 1:10, E. coli J5 diluted 1:100. The admixture is then either administered or further diluted to 1:5 with phosphate buffered saline.

Twenty six-week-old White Swiss Webster mice from SASCO, Omaha, Nebraska are used for each dilution. The mice are vaccinated with 0.1 ml IP and are challenged 14 days after the vaccination with 0.20 ml of a 10⁶ dilution of the challenge strain (USDA strain 169).

Table 5 shows the results of the experiment using bacterin alone or in combination with E. coli J5 at various dilutions.

**Table 5**

| Dilution of P. Multocida/E. coli J5 Bacterin Tested | | |
|---|---|---|
| VACCINE | UNDILUTED | 1:5 |
| J-5 Undiluted | 4/20* | 11/20 |
| J-5 1:10 | 8/20 | 17/20 |
| J-5 1:100 | 6/20 | 14/20 |
| No J-5 | 10/20 | 12/20 |

| | | |
|---|---|---|
| * No. of dead/No. challenged. | | |

### Example 7

A vaccine is prepared with E. coli J5 bacterin and Midcon Labs' Moraxella bovis (MB) as in the procedures of Example 2.

Cattle were vaccinated twice with either bacterin alone or the co-vaccine preparation of E. coli J5 bacterin and Moraxella bovis bacterin. The time lapse between the first and second vaccinations was 21 days. 25 days after the second vaccination the cattle were challenged with Moraxella bovis. The challenged cattle were examined 7, 15, 19, 29 and 40 days after challenge for visible gross ocular lesions.

Table 6 shows the results of the challenge testing.

**TABLE 6**

| DAYS AFTER CHALLENGE | MIDCON MB STANDARD | MIDCON MB WITH 25% J5 | MIDCON MB WITH 50% J5 | UNVACCINATED CONTROLS |
|---|---|---|---|---|
| 7 | 114/121* | 111/111 | 89/90 | 73/87 |
| 15 | 113/121 | 111/111 | 89/90 | 64/87 |
| 19 | 111/121 | 109/109+ | 86/90 | 50/86⁻ |
| 29 | 110/121 | 109/109 | 84/90 | 39/86 |
| 40 | 110/121 | 109/109 | 84/90 | 37/86 |

| | | | | |
|---|---|---|---|---|
| * No. of Symptom Free Animals/ No. Challenged. | | | | |
| + Two calves lost to an electrical storm. | | | | |
| ⁻ One infected calf lost to an electrical storm. | | | | |

## Claims

1. A composition effective for co-injection of an animal to enhance the immune response of said animal against a gram-negative pathogen which comprises an effective dose of
a) gram-negative bacterial cells, as source of bacterial lipopolysaccharide devoid of O-carbohydrate side-chains; and
b) a whole cell bacterin derived from said pathogen.

2. A composition as in claim 1, wherein said lipopolysaccharide is contained in cells of gram-negative bacteria.

3. A composition as in claim 2, wherein said cells are E. coli J5 or mutants thereof.

4. A composition of claim 1, wherein said pathogen is one or more selected from the group consisting of Pasteurella multocida, P. hemolytica, Escherichia coli, Bordetella bronchiseptica, Salmonella typhimurium, S. choleraesuis, S. dublin, Pseudomonas aeruginosa, Haemophilus pleuropneumoniae, H. parasuis, H. sommnus, Moraxella bovis, Treponema hyodysenteriae, Campylobacter sputurum, C. hyointestinalis, Leptospira canicola, L. grippotyphosa, L. hardjo, L. icterohaemorrhagiae, L. pomoma, and L. bratislava.

5. A composition of claim 3, wherein said pathogen is one or more selected from the group consisting of Pasteurella multocida, P. hemolytica, Escherichia coli, Bordetella bronchiseptica, Salmonella typhimurium, S. choleraesuis, S. dublin, Pseudomonas aeruginosa, Haemophilus pleuropneumoniae, H. parasuis, H. sommnus, Moraxella bovis, Treponema hyodysenteriae, Campylobacter sputurum, C. hyointestinalis, Leptospira canicola, L. grippotyphosa, L. hardjo, L. icterohaemorrhagiae, L. pomoma, and L. bratislava.

6. A composition as in claim 2, wherein said cells are concentrated to about 5 to about 50 percent by volume.

7. A composition as in claim 3, wherein said cells are concentrated to about 2-3 x 10¹⁰ cfu/ml.

## Patentansprüche

1. Zusammensetzung, die bei der Coinjektion bei Tieren zur Verbesserung der Immunantwort auf gramnegative Krankheitserreger wirksam ist und eine wirksame Dosis
a) von gramnegativen Bakterienzellen als Quelle bakterieller Lipopolysaccharide ohne O-Kohlenhydrat-Seitenketten und
b) eines aus den Krankheitserregern erhaltenen Bakterien-Vakzins aus ganzen Zellen
enthält.

2. Zusammensetzung nach Anspruch 1, wobei das Lipopolysaccharid in den Zellen gramnegativer Bakterien enthalten ist.

3. Zusammensetzung nach Anspruch 2, wobei die Zellen E. coli J5 oder Mutanten davon sind.

4. Zusammensetzung nach Anspruch 1, wobei der oder die Krankheitserreger unter Pasteurella multocida, P. haemolytica, Escherichia coli, Bordetella bronchiseptica, Salmonella typhimurium, S. choleraesuis, S. dublin, Pseudomonas aeruginosa, Haemophilus pleuropneumoniae, H. parasuis, H. sommnus, Moraxella bovis, Treponema hyodysenteriae, Campylobacter sputurum, C. hyointestinalis, Leptospira canicola, L. grippotyphosa, L. hardjo, L. icterohaemorrhagiae, L. pomoma und L. bratislava ausgewählt sind.

5. Zusammensetzung nach Anspruch 3, wobei der oder die Krankheitserreger unter Pasteurella multocida, P. haemolytica, Escherichia coli, Bordetella bronchiseptica, Salmonella typhimurium, S. choleraesuis, S. dublin, Pseudomonas aeruginosa, Haemophilus pleuropneumoniae, H. parasuis, H. sommnus, Moraxella bovis, Treponema hyodysenteriae, Campylobacter sputurum, C. hyointestinalis, Leptospira canicola, L. grippotyphosa, L. hardjo, L. icterohaemorrhagiae, L. pomoma und L. bratislava ausgewählt sind.

6. Zusammensetzung nach Anspruch 2, wobei die Zellen auf etwa 5 bis etwa 50 Vol.-% aufkonzentriert sind.

7. Zusammensetzung nach Anspruch 3, wobei die Zellen bis auf etwa 2 · 10¹⁰ bis 3 · 10¹⁰ cfu/ml aufkonzentriert sind.

## Revendications

1. Composition efficace pour co-injection à un animal, pour amplifier la réponse immunitaire dudit animal vis-à-vis d'un agent pathogène Gram négatif, laquelle comprend une dose efficace :
a) de cellules bactériennes Gram négatif, comme source de lipopolysaccharide bactérien dépourvu de chaînes latérales O-saccharidiques ; et
b) de vaccin bactérien à base de cellules entières provenant dudit agent pathogène.

2. Composition selon la revendication 1, dans laquelle ledit lipopolysaccharide est contenu dans des cellules de bactéries Gram négatif.

3. Composition selon la revendication 2, dans laquelle lesdites cellules sont E. coli J5 ou ses mutants.

4. Composition selon la revendication 1, dans laquelle ledit agent pathogène est un ou plusieurs agents sélectionnés dans le groupe constitué par Pasteurella multocida, P. hemolytica, Escherichia coli, Bordetella bronchiseptica, Salmonella typhimurium, S. choleraesuis, S. dublin, Pseudomonas aeruginosa, Haemophilus pleuropneumoniae, H. parasuis, H. sommnus, Moraxella bovis, Treponema hyodysenteriae, Campylobacter sputurum, C. hyointestinalis, Leptospira canicola, L. grippotyphosa, L. hardjo, L. icterohaemorrhagiae, L. pomoma et L. bratislava.

5. Composition selon la revendication 3, dans laquelle ledit agent pathogène est un ou plusieurs agents sélectionnés dans le groupe constitué par Pasteurella multocida, P. hemolytica, Escherichia coli, Bordetella bronchiseptica, Salmonella typhimurium, S. choleraesuis, S. dublin, Pseudomonas aeruginosa, Haemophilus pleuropneumoniae, H. parasuis, H. sommnus, Moraxella bovis, Treponema hyodysenteriae, Campylobacter sputurum, C. hyointestinalis, Leptospira canicola, L. grippotyphosa, L. hardjo, L. icterohaemorrhagiae, L. pomoma et L. bratislava.

6. Composition selon la revendication 2, dans laquelle la concentration desdites cellules est entre environ 5 et environ 50% en volume.

7. Composition selon la revendication 3, dans laquelle la concentration desdites cellules est environ 2-3x10¹⁰ unités formant colonie/ml.
